# EUROPÄISCHE PATENTSCHRIFT

(11) **EP 1 930 080 B1**
(45) Veröffentlichungstag und Bekanntmachung des Hinweises auf die Patenterteilung: **06.11.2013**
(21) Anmeldenummer: 07023513.0
(22) Anmeldetag: 05.12.2007
(51) Int. Cl.: B03B 9/06, C10L 3/08, C12P 5/02

(54) **Verfahren zur Perkolataufbereitung und Perkolataufbereitungsanlage**
Method for preparing percolate and percolate preparation plant
Procédé de préparation de percolat et installation de préparation de percolat

(30) Priorität: 08.12.2006 DE 102006058419
(43) Veröffentlichungstag der Anmeldung: 11.06.2008
(73) Patentinhaber: Zweckverband Abfallbehandlung Kahlenberg, 77975 Ringsheim (DE)
(72) Erfinder: Berkenbusch, Frank, 31683 Obernkirchen (DE); Drinkuth, Katharina, 32423 Minden (DE); Gibis, Georg, 77975 Ringsheim (DE); Sauter, Tobias, 77977 Rust (DE)
(74) Vertreter: KEIL & SCHAAFHAUSEN Patentanwälte

(56) Entgegenhaltungen:
- EP-A2- 0 937 504
- WO-A1-2006/089766
- WO-A2-2004/083125
- DE-A1- 19 602 489
- DE-A1-102005 026 027

## Beschreibung

Die Erfindung betrifft ein Verfahren und eine Anlage zur Perkolataufbereitung, welche insbesondere innerhalb einer Abfallaufbereitungsanlage zum Abscheiden von bei der Umsetzung der organischen Inhaltsstoffe in Biogas unerwünschten Stoffen eingesetzt wird.

In Dokument WO 2004/083125 A2 wird ein Verfahren zur Perkolataufbereitung, insbesondere von Restmüll, beschrieben. Der Anteil an Feststoffen wird durch eine Ultrafiltration verringert, während Feststoffe, Schwimm- und Faserstoffe durch eine Siebstufe abgetrennt werden. In dem beschriebenen Verfahren wird sowohl eine mechanische als auch eine biologische Aufbereitung von Müll durchgeführt, wobei organische Bestandteile durch Zuführung von Prozesswasser gelöst und/oder ausgetrieben werden. Die mit Organik beladene Flüssigkeit wird zum anaeroben Abbau einer Biogasanlage zugeführt.

Die DE 196 02 489 A1 beschreibt ein Verfahren und eine Vorrichtung zur biologischen Behandlung von organischen Materialien. Die sich in den Materialien befindlichen organischen Substanzen werden durch Zufuhr von Frischluft aerob zersetzt, während das Material durchmischt und transportiert wird. Bei dem aeroben Zersetzungsprozess werden die löslichen organischen und anorganischen Substanzen sowie die wasserlöslichen Fettsäuren durch Berieselung mit einer Auswaschflüssigkeit aus den Materialien ausgewaschen.

Die WO 20061089766 A1 beschreibt ebenfalls ein Verfahren zur biologischen Aufbereitung von Organik enthaltenden Abfallstoffen und einen Reaktor zur Durchführung des Verfahrens. Bei dem Verfahren wird dem Reaktor, in dem sich die biologischen Abfallstoffe befinden, Prozesswasser zugegeben, wodurch wasserlösliche Bestandteile aus dem Abfallstoff herausgewaschen werden. Das derartig mit organischen Bestandteilen beladenen Prozesswasser wird abgezogen und in einer Biogasanlage aufbereitet.

Die DE 10 2005 026 027 A1 offenbart ein Verfahren zur Behandlung von Abfall mit organischen Bestandteilen, einen Stofflöser zum Lösen organischer Bestandteile aus dem Abfall, einen geeigneten Reaktor zur Durchführung einer Hydrolyse der aus dem Stofflöser abgezogenen Suspension und eine Abfallaufbereltungsanlage zur Verwendung in dem Verfahren.

In mechanisch-biologischen Abfallbehandlungsanlagen wird Restabfall, insbesondere Haushaltsabfall einschließlich Bioabfällen, einer Behandlung mit dem Ziel der weitgehenden Massenreduzierung und der Verwertung sowie der Erzeugung von Biogas unterzogen. Eine derartige Anlage sowie ein entsprechendes Verfahren sind aus der EP 0 937 504 B1 bekannt. Weiter ist es bspw. aus der DE 20 2005 019 033 U1 bekannt, die organischen Bestandteile des Abfalls in einem sogenannten Perkolator durch Berleselung des Abfalls in die wässrige Phase (Perkolat) zu überführen, wobei das Perkolat dann einem Vergärungsreaktor zur Vergärung der nativ-organischen Bestandteile zu Biogas zugeführt werden kann.

Nach dem Auswaschen, Aufschließen (Hydrolysieren) und Homogenisieren des Restabfalls in dem Perkolator enthält das Perkolat noch Stoffe, welche in der Vergärungsstufe unerwünscht sind.

Es ist daher eine Aufgabe der vorliegenden Erfindung, ein Verfahren zur Perkolataufbereitung sowie eine Abfallaufbereitungsanlage bereit zu stellen, welche eine wirtschaftliche Abfallbehandlung und eine möglichst vereinfachte Trennung von Stoffen in verwertbare Fraktionen und Deponiegut ermöglichten.

Diese Aufgabe wird erfindungsgemäß mit einem Verfahren zur Perkolataufbereitung gelöst, bei welchem Perkolat, welches durch Auswaschen von Restabfall erzeugt wird, einer Aufbereitungsstufe zugeführt wird, die eine Schwimm- und Sinkstoffabscheidung und eine Faserstoffabscheidung aufweist, in welchen Schwimm- und Sinkstoffe sowie Faserstoffe abgeschieden werden, bevor das derart aufbereitete Perkolat wenigstens einer Vergärungsstufe zur Vergärung nativ-organischer Bestandteile zu Biogas zugeführt wird. Bei dem erfindungsgemäßen Verfahren wird das Perkolat in der Aufbereitungsstufe zunächst einem Trommelsiebsystem zur Grobstoffabscheidung zugeführt, in welchem Grobstoffe mit einer Größe von über etwa 3 mm bis über etwa 20 mm abgeschieden werden. Die erfindungsgemäße Perkolataufbereitung ermöglicht es, die Vergärungsstufe von einer Vergärung nicht zugänglichen Feststoffen zu entlasten.

Es wird bevorzugt, wenn in der Grobstoffabscheidung Perkolatinhaltsstoffe mit einer Größe von über etwa 6 mm abgesiebt werden. Diese Grobstoffe bilden einen wesentlichen Bestandteil der abzutrennenden Feststoffe und liegen in Form von beispielsweise Textilien, Kunststofffetzen sowie mineralischen Stoffen wie Steinen, Keramik Metallen und Glasscherben vor.

Eine besonders effiziente Grobstoffabscheidung kann dadurch erreicht werden, dass das Perkolat in der Aufbereitungsstufe insbesondere mittels eines Spiralförderers einem Trommelsiebsystem zur Grobstoffabscheidung zugeführt wird. Das aufzubereitende Perkolat kann bspw. mittels eines Spiralförderers einem innen beschickten und vorzugsweise horizontal liegenden Trommelsieb kontinuierlich zugefügt werden. Dabei wird es bevorzugt, wenn Spiralförderer abwurfseitig so an das Trommelsieb angebunden sind, dass sie weit in das Trommelsieb hineinragen, um ein betriebssicheres Abscheiden von Feststoffen zu gewährleisten. Das Trommelsieb kann gelocht, geschlitzt oder als Spaltsieb ausgeführt sein. Um eine Abreinigung der Trommelaußenfläche zu erreichen, sind vorzugsweise außen liegende Spritzleisten und/oder ein insbesondere außen liegendes Schabersystem bzw. Bürsten oder Kammsysteme an dem Trommelsieb vorgesehen. Weiter kann das Trommelsiebsystem geruchsgekapselt aus-geführt sein. Der Feststoffaustrag aus dem Trommelsiebsystem erfolgt vorzugsweise über eine innen angeordnete bspw. segmentförmige Spirale.

Nach einer weiteren Ausführungsform der Erfindung werden in der Schwimm-und Sinksfoffabscheidung der Aufbereitungsstufe Schwimmschlamm oder dergleichen aufschwimmende Feststoffe über einen Beckenräumer und Sediment über einen Sandfang aus dem Perkolat abgeschieden. Die durch Sedimentation abgeschiedenen Schwerstoffe wie Sand, Inertstoffe und Sinkstoffe können durch den Sandfang mit einer Abscheideleistung von etwa 95% bezogen auf eine Partikelgröße von etwa 0,2 mm aus dem Perkolat abgeschieden werden.

Dabei wird es besonders bevorzugt, wenn der Sandfang eine waagerecht im unteren Bereich montierte Sandräumerschnecke aufweist, die das Sediment entgegen der Strömungsrichtung ausräumt und in eine Steilförderspirale übergibt. Hierdurch kann das Sediment in der Steilförderschnecke statisch entwässert werden. Weiter ist vorzugsweise eine in Bodennähe des Sandfangs angeordnete Sandfangbelüftung vorgesehen, die die Abtrennung von am Sand anhaftender Organik während der Sedimentation unterstützt. Der nach einer bevorzugten Ausführungsform in dem Sandfang angeordnete Schwimmdeckenräumer kann über die Gesamtbreite des Sandfangs eine automatische und vollständige Entfernung der aufschwimmenden Feststoffe von der Wasseroberfläche übernehmen. Der Räumer ist dabei vorzugsweise als ein Kettenumlaufräumer mit Paddeln ausgestaltet. Der Schwimmschlamm kann mit einer Abwurfschurre in eine Entwässerungsschnecke mit einem durchgehenden Spaltsiebboden übergeben werden. Dabei wird es bevorzugt, wenn auch der Sandfang geruchsdicht gekapselt ist.

In Weiterbildung des Erfindungsgedankens ist es vorgesehen, dass das Perkolat in der Faserstoffabscheidung einem Trommelsiebsystem mit einer effektiven Siebweite von etwa 0,2 mm bis etwa 1 mm, insbesondere etwa 0,5 mm, zugeführt wird. Das Trommelsiebsystem kann dabei eine horizontal gelagerte und tangential von innen angeströmte Siebtrommel aufweisen und als ein Spaltsieb mit radialen oder insbesondere axialen Spalten ausgebildet sein. Zur Reinigung der Trommelaußenfläche ist das Trommelsiebsystem vorzugsweise mit einer außen liegenden Nieder- und/oder Hochdruckspritzeinrichtung ausgerüstet. Die abgetrennten Faserstoffe werden aus dem Trommelsiebsystem vorzugsweise über eine innen angeordnete bspw. segmentförmige Spirale ausgetragen und können über einen Fallschacht an ein Schneckenfördersystem mit durchgehendem Spaltsiebboden und einseitiger Schneckenlagerung übergeben werden. In diesem Schneckenfördersystem werden die Faserstoffe mechanisch entwässert und gepresst und können über ein Spiralfördersystem weitertransportiert werden.

Erfindungsgemäß ist das Verfahren derart ausgestaltet, dass das Perkolat in der Aufbereitungsstufe zunächst der Grobstoffabscheidung, dann der Schwimm-und Sinkstoffabscheidung und schließlich der Faserstoffabscheidung unterworfen wird. Hierbei wird es bevorzugt, wenn das Trommelsieb der Grobstoffabscheidung dem Sandfang direkt vorgeschaltet ist, so dass der Siebdurchlauf in freiem Gefälle dem Sandfang zuläuft. Die abgetrennten Feststoffe können dabei kontinuierlich einem Spiralpressensystem zugeführt werden. Der getauchte Ablauf des sogenannten Klarlaufes aus dem Sandfang wird vorzugsweise über eine Rohrleitung dem als Trommelsieb ausgeführten Faserstoffabscheider zugeführt.

Der zur Perkolaterzeugung eingesetzte Restabfall wird vorzugsweise mechanisch vorbehandelt, wobei vor der Perkolation Grobgut, d.h. Feststoffe mit einer Größe von beispielsweise über etwa 150 mm, eisenhaltige Metalle, Störstoffe, Mineralien und/oder Schwerstoffe aus dem Restabfall mechanisch abgetrennt werden. Die Abtrennung kann dabei beispielsweise durch Siebung, Sichtung und/oder Magnetscheidung erfolgen. Hierdurch können bereits vor der Aufbereitungsstufe Anteile des angelieferten Abfalls, beispielsweise etwa 10% des Abfalls, abgetrennt werden.

Die Auswaschung und Hydrolyse in dem der Aufbereitungsstufe vorgeschalteten Perkolator erfolgt vorzugsweise durch Berieselung des Restabfalls mit Wasser.

In Weiterbildung des Erfindungsgedankens ist es vorgesehen, dass die in der Aufbereitungsstufe aus dem Perkolat abgeschiedenen Stoffe beispielsweise mechanisch entwässert und danach thermisch oder insbesondere biologisch getrocknet werden. Die mechanische Entwässerung kann dabei durch Spiralpressen, Entwässerungsschnecken und/oder Schneckenpressen erfolgen. Anschließend können die entwässerten und getrockneten Stoffe einer mit vorzugsweise mechanischen Stofftrennung zugeführt werden. Der Trockenaustrag, der bei dem erfindungsgemäßen Verfahren beispielsweise etwa 40% der ursprünglich angelieferten Abfallmasse beträgt, kann dabei in bspw. energetisch verwertbare Fraktionen und eine ablagerungsfähige Inertfraktion (Deponiegut) getrennt werden.

Der Frischwasserverbrauch kann bei dem erfindungsgemäßen Verfahren minimiert werden, wenn das Presswasser der mechanischen Entwässerung und das Abwasser der Vergärungsstufe und der Aufbereitungsstufe einer Prozesswasseraufbereitung unterzogen werden und zumindest teilweise wieder dem Verfahren zur Erzeugung des Perkolats aus dem Restabfall und/oder als Spülwasser zugeführt werden. Es ist auch möglich, das Filtrat aus dem Faserstoffabscheider zu sammeln und als Spritzwasser für den Grobstoffabscheider zu verwenden. Soweit die anfallenden Prozesswässer nicht prozessintern eingesetzt werden, werden diese vor der Einleitung in die Kanalisation in einer Reinigungsanlage behandelt.

Das erfindungsgemäße Verfahren nach einer Ausführungsformist derart ausgelegt, dass der zugeführte Restabfall vor der Perkolation insbesondere eine Eingangsschüttdichte von etwa 0,35 bis etwa 0,5 Mg/m³ und eine Anlieferfeuchte von etwa 30 bis 50% aufweisen kann.

Die der Erfindung zugrunde liegende Aufgabe wird weiter durch eine Perkolataufbereitungsanlage gelöst, die einem Perkolator nachgeschaltet und einer Stufe zur biologischen Umsetzung, d. h. einer Vergärungsstufe, vorgeschaltet ist und ein erstes Trommelsiebsystem zur Abscheidung von Grobstoffen mit einer Größe von über etwa 6 mm, eine diesem nachgeschaltete Sandfangeinrichtung und ein dieser nachgeschaltetes zweites Trommelsiebsystem zur Abscheidung von Faserstoffen aufweist.

Vorzugsweise ist in der Perkolataufbereitungsanlage als erstes Trommelsiebsystem zur Abscheidung von Grobstoffen mit einer Größe von über etwa 6 mm wenigstens ein von innen beschicktes Trommelsieb vorgesehen, das zur Reinigung der Trommelaußenfläche mit einer außen liegenden Spritzleiste und/oder einem insbesondere außen liegenden Schaber-, Bürsten- oder Kammsystem ausgestattet ist.

Weiter wird es bevorzugt, wenn die Perkolataufbereitungsanlage als Sandfangsystem wenigstens einen Sandfang mit einer Sandfangbelüftung zur Abtrennung von organischen Stoffen von dem Sediment und einen Räumer zum Entfernen von aufschwimmenden Feststoffen oder Schwimmschlamm aufweist. Der Räumer kann ein Kettenumlaufräumer mit Paddeln sein.

Um Faserstoffe aus dem Perkolat abzuscheiden, weist die Anlage als zweites Trommelsiebsystem vorzugsweise wenigstens ein von innen beschicktes Trommelsieb mit einer effektiven Siebweite von etwa 0,5 mm auf, das mit einer außen liegenden Nieder- und/oder Hochdruckspritzeinrichtung versehen ist.

Nach einer bevorzugten Ausführungsform der Erfindung ist die Perkolataufbereitungsanlage derart ausgestaltet, dass das Trommelsieb zur Abscheidung von Grobstoffen, der Sandfang und das Trommelsieb zur Abscheidung von Faserstoffen gekapselt sind. Hierdurch wird zum einen vermieden, dass in der Umgebung der Anlage eine Geruchsbelästigung oder eine gesundheitsgefährdende Luftbelastung auftritt. Zum anderen können die Abluftströme erfasst und einem geeigneten Reinigungsverfahren zugeführt werden. Dabei ist es auch möglich, verschieden belastete Abluftströme getrennt zu erfassen und geeigneten Reinigungsverfahren zuzuführen. Dies kann beispielsweise derart erfolgen, dass die Abluftströme der ersten, mechanischen Aufbereitungsstufe, der biologischen Umsetzung und der beispielsweise biologischen Trocknung, welche in der Regel eher schwach belastet sind, sowie die stärker belasteten Prozessabluftströme der biologischen Verfahrenseinheiten getrennt erfasst und gereinigt werden.

Um einen höheren Durchsatz der Anlage zu ermöglichen und gleichzeitig bei Reparatur- und Wartungsarbeiten zumindest einen eingeschränkten Betrieb fortführen zu können, wird es bevorzugt, wenn die Perkolataufbereitungsanlage zwei Trommelsiebe zur Abscheidung von Grobstoffen, zwei Sandfänge und zwei Trommelsiebe zur Abscheidung von Faserstoffen aufweist. Bei dieser Ausgestaltung der Perkolataufbereitungsanlage können auch mehrere dieser vorgeschaltete Perkolatoren vorgesehen sein.

Die erfindungsgemäße Perkolataufbereitungsanlage kann Bestandteil einer Abfallaufbereitungsanlage sein, die eine mechanische Aufbereitungsstufe zur Abtrennung von Grobgut, eisenhaltigen Metallen, Störstoffen, Mineralien und Schwerstoffen, eine dieser nachgeschaltete Stufe zur Perkolation, Perkolataufbereitung und zur biologischen Umsetzung, eine Stufe zur insbesondere biologischen Trocknung der in der Perkolataufbereitung aus dem Perkolat abgeschiedenen Stoffe und eine der Stufe zur Trocknung nachgeschalteten Stufe zur mechanischen Stofftrennung aufweist. Mit dieser Abfallaufbereitungsanlage ist es möglich, Resthausabfälle und Bioabfälle effizient zu behandeln, so dass die Abfallmenge reduziert wird, wobei verwertbare Fraktionen abgetrennt sowie die Erzeugung von Biogas ermöglicht wird.

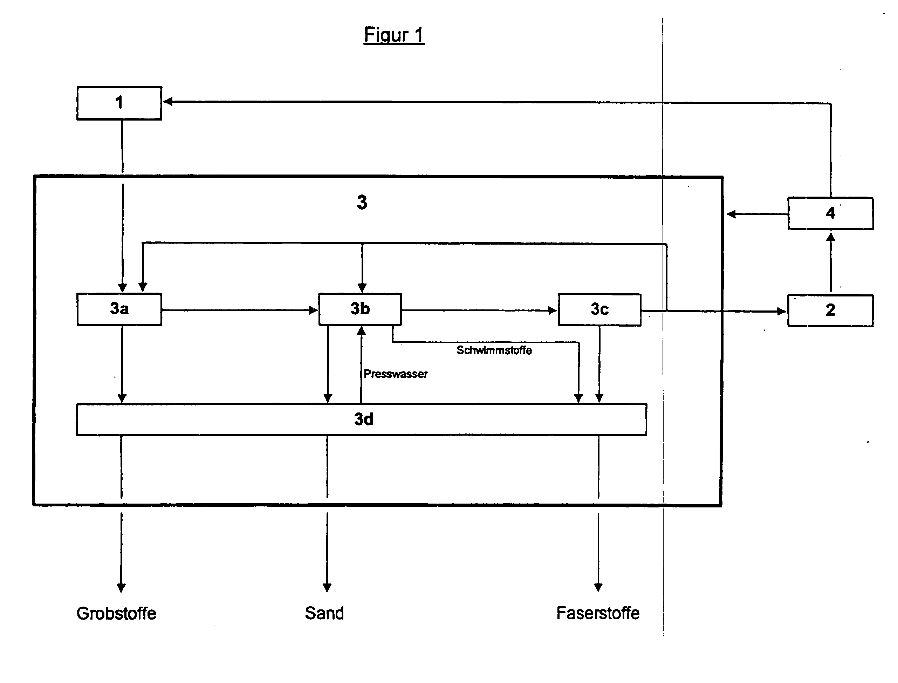

Weiterbildungen, Vorteile und Anwendungsmöglichkeiten der vorliegenden Erfindung ergeben sich auch aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Die einzige Figur zeigt schematisch ein Blockschaltbild einer Abfallaufbereitungsanlage mit einer erfindungsgemäßen Perkolataufbereitung.

In der Abfallaufbereitungsanlage wird Restabfall, d. h. Haushaltsabfälle vorzugsweise einschließlich Bioabfällen zunächst mechanisch aufbereitet und dann einer biologischen Umsetzung zugeführt. Hierzu wird der mechanisch vorbehandelte Restabfall zunächst in einem Perkolator 1 mit Wasser berieselt und hierdurch in eine wässrige Phase überführt.

Bevor aus dem Perkolat in einem Gärreaktor 2 Biogas erzeugt wird, wird das Perkolat in einer Aufbereitungsstufe 3 von Stoffen gereinigt, welche für die Vergärung nicht geeignet sind. Hierzu wird das Perkolat in ein dem Perkolator 1 nachgeschaltetes Trommelsiebsystem 3a (insbesondere NOGGERATH Multi-Drum^{®}) zugeführt. Das Trommelsiebsystem 3a ist dabei durch ein horizontal liegendes Trommelsieb gebildet, dem das Perkolat mittels eines Spiralförderers kontinuierlich innen zugeführt wird. Dabei sind die Spiralförderer abwurfseitig so an das Trommelsieb angebunden, dass sie weit in dieses hineinragen. Die beispielsweise gelocht, geschlitzt oder als Spaltsieb ausgebildete Trommel weist dabei außen liegende Spritzleisten und ein Schabersystem zur Abreinigung der Trommelaußenfläche auf.

Das Trommelsieb 3a ist einem als Sandfang ausgebildeten Abscheider 3b für Schwimm- und Sinkstoffe direkt vorgeschaltet. Der Siebdurchlauf des Trommelsiebes kann dabei in freiem Gefälle in den Sandfang laufen. Die in dem Sandfang 3b zu Boden sinkenden Stoffe werden entgegen der Strömungsrichtung durch eine Sandräumschnecke ausgeräumt, während ein Räumer über die Gesamtbreite des Sandfanges die automatische und vollständige Entfernung der Feststoffe von der Wasseroberfläche übernimmt.

Dem Sandfang 3b ist ein Faserstoffabscheider 3c nachgeschaltet, wobei der getauchte Ablauf des Klarlaufes aus dem Sandfang über eine Rohrleitung dem als Trommelsieb 3a (insbesondere NOGGERATH Multi-Drum^{®}) ausgeführten Faserstoffabscheider 3c zugeführt wird. Das Trommelsieb 3a ist horizontal gelagert und wird durch den Klarlauf aus dem Sandfang tangential von innen angeströmt, wobei der Faserstoffabscheider 3c eine effektive Siebweite von etwa 0,5 mm besitzt. Durch außen liegende Nieder- und/oder Hochdruckspritzeinrichtungen kann die Trommelaußenfläche gereinigt werden.

Feststoffe aus dem der Grobstoffabscheidung dienenden ersten Trommelsiebsystems 3a werden kontinuierlich einem Spiralpressensystem zur mechanischen Entwässerung zugeführt. Auch das aus dem Sandfang 3b ausgetragene Sediment wird beispielsweise über eine Steilförderschnecke statisch entwässert. Der durch den Räumer von der Wasseroberfläche des Sandfangs abgezogene Schwimmschlamm wird über eine Abwurfschurre in eine Entwässerungsschnecke mit durchgehendem Spaltsiebboden übergeben. Auch die in der Faserstoffabscheidung 3c abgetrennten Faserstoffe werden in ein Schneckenfördersystem mit durchgehendem Spaltsiebboden und beispielsweise einseitiger Schneckenlagerung übergeben. Diese Komponenten bilden zusammen eine mechanische Entwässerungsstufe 3d für die aus dem Perkolat abgeschiedenen Stoffe.

Das Presswasser der mechanischen Entwässerungsstufe 3d kann entweder in die Perkolataufbereitungsstufe 3 zurückgeführt werden, beispielsweise als Spritzwasser für den Grobstoffabscheider, oder in eine Prozesswasser-Aufbereitungsstufe geleitet werden.

Der Prozesswasser-Aufbereitungsstufe 4 wird u.a. das Abwasser des Vergärreaktors 2 zugeleitet, wobei das gereinigte Abwasser entweder in die Kanalisation abgeleitet werden kann oder zumindest zum Teil als Prozesswasser beispielsweise dem Perkolator 1 zugeführt oder als Brauchwasser für den internen Gebrauch genutzt werden kann.

Die in der mechanischen Entwässerungsstufe 3d entwässerten Reststoffe, beispielsweise Grob- oder Faserstoffe, können ggf. zusammen mit anderen Reststoffen einer in der Figur nicht näher dargestellten beispielsweise thermischen oder biologischen Trocknungsstufe oder einer Rotte zugeführt werden. Nach diesem Behandlungsschritt können diese Stoffe in verschiedene verwertbare Fraktionen und Deponiegut getrennt werden. Die Sandfraktion aus der mechanischen Entwässerungsstufe 3d ist ohne weitere Nachbehandlung als Deponiegut ablagerbar.

Das in dem Vergärreaktor 2 anfallende Biogas wird zunächst einer Gassicherheitseinrichtung zugeleitet, bevor es zur weiteren Verwendung beispielsweise in einem Blockheizkraftwerk oder dergleichen abgegeben wird. Der Sedimentschlamm des Vergärreaktors 2 wird nach einer Entwässerung und Trocknung entsorgt.

## Patentansprüche

1. Verfahren zur Perkolataufbereitung, insbesondere als Bestandteil eines mechanischen und/oder biologischen Abfallbehandlungsverfahrens, bei welchem Perkolat, welches durch Auswaschen von Restabfall, insbesondere Haushaltsabfall, erzeugt wird, einer Aufbereitungsstufe (3) zugeführt wird, die eine Schwimm- und Sinkstoffabscheidung (3b) und eine Faserstoffabscheidung (3c) aufweist, in welchen Schwimm- und Sinkstoffe und Faserstoffe abgeschieden werden, bevor das derart aufbereitete Perkolat wenigstens einer Vergärungsstufe (2) zur Vergärung nativ-organischer Bestandteile zu Biogas zugeführt wird, **dadurch gekennzeichnet, dass** das Perkolat in der Aufbereitungsstufe (3) zunächst einem Trommelsiebsystem (3a) zur Grobstoffabscheidung zugeführt wird, in welchem Grobstoffe mit einer Größe von über etwa 3 mm bis über etwa 20 mm abgeschieden werden.

2. Verfahren nach Anspruch 1, **dadurch gekennzeichnet, dass** in der Grobstoffabscheidung (3a) Perkolatinhaltsstoffe mit einer Größe von über etwa 6 mm abgesiebt werden.

3. Verfahren nach Anspruch 1 oder 2, **dadurch gekennzeichnet, dass** das Perkolat in der Aufbereitungsstufe (3) mittels eines Spiralförderers dem Trommelsiebsystem (3a) zugeführt wird.

4. Verfahren nach einem der vorhergehenden Ansprüche, **dadurch gekennzeichnet, dass** in der Schwimm- und Sinkstoffabscheidung (3b) Schwimmschlamm über einen Beckenräumer und Sediment über einen Sandfang aus dem Perkolat abgeschieden werden.

5. Verfahren nach einem der vorhergehenden Ansprüche, **dadurch gekennzeichnet, dass** das Perkolat in der Faserstoffabscheidung (3c) einem Trommelsiebsystem mit einer effektiven Siebweite von etwa 0,2 mm bis etwa 1 mm, insbesondere etwa 0,5 mm, zugeführt wird.

6. Verfahren nach einem der vorhergehenden Ansprüche, **dadurch gekennzeichnet, dass** das Perkolat nach der Grobstoffabscheidung (3a) insbesondere in freiem Gefälle dem mit einem Beckenräumer ausgestatteten Sandfang (3b) der Schwimm- und Sinkstoffabscheidung zugeführt wird, dem die Faserstoffabscheidung (3c) nachgeschaltet ist.

7. Verfahren nach einem der vorhergehenden Ansprüche, **dadurch gekennzeichnet, dass** der Aufbereitungsstufe (3) eine Stufe zur mechanischen Abtrennung von Grobgut mit einer Größe von über etwa 150 mm, eisenhaltigen Metallen, Störstoffen, Mineralien und/oder Schwerstoffen aus dem Restabfall vorgeschaltet ist.

8. Verfahren nach einem der vorhergehenden Ansprüche, **dadurch gekennzeichnet, dass** das Perkolat durch Berieselung des Restabfalls mit Wasser erzeugt wird.

9. Verfahren nach einem der vorhergehenden Ansprüche, **dadurch gekennzeichnet, dass** die in der Aufbereitungsstufe (3) aus dem Perkolat abgeschiedenen Stoffe mechanisch entwässert und/oder biologisch getrocknet werden.

10. Verfahren nach Anspruch 9, **dadurch gekennzeichnet, dass** die entwässerten und getrockneten Stoffe einer mechanischen Stofftrennung zugeführt werden.

11. Verfahren nach einem der vorhergehenden Ansprüche, **dadurch gekennzeichnet, dass** das Presswasser der mechanischen Entwässerung und/oder das Abwasser der Vergärungsstufe (2) und/oder der Aufbereitungsstufe (3) einer Prozesswasseraufbereitung (4) unterzogen und zumindest teilweise wieder dem Verfahren zur Erzeugung des Perkolats aus dem Restabfall und/oder als Spülwasser zugeführt wird.

12. Verfahren nach einem der vorhergehenden Ansprüche, **dadurch gekennzeichnet, dass** der dem Verfahren zugeführte Restabfall vor der Perkolation eine Eingangsschüttdichte von etwa 0,35 bis etwa 0,5 Mg/m³ und eine Anlieferfeuchte von etwa 30 bis 50% aufweist.

13. Perkolataufbereitungsanlage, insbesondere zur Durchführung eines Verfahrens nach einem der vorhergehenden Ansprüche, die einem Perkolator (1) einer Abfallaufbereitungsanlage nachgeschaltet und einer Stufe zur biologischen Umsetzung und/oder Vergärung (2) vorgeschaltet ist, dadurch gekennzeichnen, dass diese ein erstes Trommelsiebsystem (3a) zur Abscheidung von Grobstoffen mit einer Größe von über etwa 6 mm, eine diesem nachgeschaltete Sandfangeinrichtung (3b) und ein dieser nachgeschaltetes zweites Trommelsiebsystem (3c) zur Abscheidung von Faserstoffen aufweist.

14. Perkolataufbereitungsanlage nach Anspruch 13, **dadurch gekennzeichnet, dass** das erste Trommelsiebsystem (3a) wenigstens ein von innen beschicktes Trommelsieb zur Abscheidung von Grobstoffen mit einer Größe von über etwa 6 mm mit einer außen liegenden Spritzleiste und/oder einem Schaber-, Bürsten- oder Kammsystem zur Reinigung der Trommelaußenfläche aufweist.

15. Perkolataufbereitungsanlage nach Anspruch 13 oder 14, **dadurch gekennzeichnet, dass** die Sandfangeinrichtung (3b) wenigstens einen Sandfang mit einer Sandfangbelüftung zur Abtrennung von organischen Stoffen von dem Sediment und einen Räumer zum Entfernen von aufschwimmenden Feststoffen oder Schwimmschlamm aufweist.

16. Perkolataufbereitungsanlage nach einem der Ansprüche 13 bis 15, **dadurch gekennzeichnet, dass** das zweite Trommelsiebsystem (3c) wenigstens ein von innen beschicktes Trommelsieb mit einer effektiven Siebweite von etwa 0,5 mm zur Faserstoffabscheidung mit einer außen liegenden Nieder- und/oder Hochdruckspritzeinrichtung aufweist.

17. Perkolataufbereitungsanlage nach einem der Ansprüche 13 bis 16, **dadurch gekennzeichnet, dass** das Trommelsieb (3a) zur Abscheidung von Grobstoffen, der Sandfang (3b) und das Trommelsieb (3c) zur Abscheidung von Faserstoffen geruchsgekapselt sind.

18. Perkolataufbereitungsanlage nach einem der Ansprüche 13 bis 17, **dadurch gekennzeichnet, dass** zwei Trommelsiebe zur Abscheidung von Grobstoffen, zwei Sandfänge und zwei Trommelsiebe zur Abscheidung von Faserstoffen vorgesehen sind.

19. Abfallaufbereitungsanlage mit
- einer ersten, mechanischen Aufbereitungsstufe zur Abtrennung von Grobgut, eisenhaltigen Metallen, Störstoffen, Mineralien und Schwerstoffen,
- einer dieser nachgeschalteten Stufe, die wenigstens einen Perkolator (1), wenigstens eine Perkolataufbereitungsanlage (3) nach einem der Ansprüche 13 bis 18 und wenigstens einen Vergärreaktor (2) aufweist,
- einer Stufe (6) zur thermischen oder biologischen Trocknung der in der Perkolataufbereitung (3) aus dem Perkolat abgeschiedenen Stoffe und
- einer der Stufe (6) zur thermischen oder biologischen Trocknung nachgeschalteten Stufe zur mechanischen Stofftrennung.

## Claims

1. A method for preparing percolate, in particular as part of a mechanical and/or biological waste treatment process, in which percolate produced by washing out residual waste, in particular household waste, is fed to a preparation stage (3) which has a floating and suspended material separation system (3b) and a fibrous material separation system (3c), in which floating and suspended material and fibrous material is separated out before the percolate prepared in this manner is fed to at least one fermentation stage (2) for fermenting native organic constituents to form biogas, **characterised in that** the percolate in the preparation stage (3) is fed first to a drum screen system (3a) for separating out coarse material, in which coarse material with a size of over approximately 3 mm to over approximately 20 mm is separated out.

2. The method according to Claim 1, **characterised in that** in the coarse material separation system (3a) percolate contents with a size of over approximately 6 mm are screened out.

3. The method according to Claim 1 or 2, **characterised in that** the percolate in the preparation stage (3) is fed to the drum screen system (3a) by means of a spiral conveyor.

4. The method according to one of the preceding claims, **characterised in that** in the floating and suspended material separation system (3b), scum is separated out from the percolate by means of a basin scraper and sediment is separated out from the percolate by means of a sand trap.

5. The method according to one of the preceding claims, **characterised in that** the percolate in the fibrous material separation system (3c) is fed to a drum screen system with an effective screen size of approximately 0.2 mm to approximately 1 mm, in particular approximately 0.5 mm.

6. The method according to one of the preceding claims, **characterised in that**, downstream of the coarse material separation system (3a), the percolate is fed in particular in a free downward gradient to the sand trap (3b) of the floating and suspended material separation system, which is equipped with a basin scraper and downstream of which the fibrous material separation system (3c) is situated.

7. The method according to one of the preceding claims, **characterised in that** a stage for mechanical separation of coarse material with a size of over approximately 150 mm, iron-containing metals, foreign materials, minerals and/or heavy materials from the residual waste is situated upstream of the preparation stage (3).

8. The method according to one of the preceding claims, **characterised in that** the percolate is produced by irrigating the residual waste with water.

9. The method according to one of the preceding claims, **characterised in that** the material separated out of the percolate in the preparation stage (3) is mechanically dehydrated and/or biologically dried.

10. The method according to Claim 9, **characterised in that** the dehydrated and dried material is fed to a mechanical material separation system.

11. The method according to one of the preceding claims, **characterised in that** the press water of the mechanical dehydration system and/or the waste water of the fermentation stage (2) and/or the preparation stage (3) is subjected to process water preparation (4) and at least partially fed back to the process for producing the percolate from the residual waste and/or as rinsing water.

12. The method according to one of the preceding claims, **characterised in that** the residual waste fed to the process upstream of percolation has an input bulk density of approximately 0.35 to approximately 0.5 mg/m³ and a delivered moisture content of approximately 30 to 50%.

13. A percolate preparation installation, in particular for carrying out a method according to one of the preceding claims, which is situated downstream of a percolator (1) of a waste preparation installation and upstream of a stage for biological reaction and/or fermentation (2), **characterised in that** it has a first drum screen system (3a) for separating out coarse material with a size of over approximately 6 mm, a sand trap device (3b) situated downstream of the latter, and a second drum screen system (3c) for separating out fibrous material situated downstream of the sand trap device.

14. The percolate preparation installation according to Claim 13, **characterised in that** the first drum screen system (3a) has at least one drum screen which is loaded from the inside for separating out coarse material with a size of over approximately 6 mm having an outer spray bar and/or a ductor, brush or comb system for cleaning the drum outer surface.

15. The percolate preparation installation according to Claim 13 or 14, **characterised in that** the sand trap device (3b) has at least one sand trap with sand trap ventilation for separating out organic material from the sediment and a scraper for removing floating solids or scum.

16. The percolate preparation installation according to one of Claims 13 to 15, **characterised in that** the second drum screen system (3c) has at least one drum screen which is loaded from the inside and has an effective screen size of approximately 0.5 mm for separating out fibrous material with an outer low- or high-pressure spray device.

17. The percolate preparation installation according to one of Claims 13 to 16, **characterised in that** the drum screen (3a) for separating out coarse material, the sand trap (3b) and the drum screen (3c) for separating out fibrous material are odour-encapsulated.

18. The percolate preparation installation according to one of Claims 13 to 17, **characterised in that** two drum screens for separating out coarse material, two sand traps and two drum screens for separating out fibrous material are provided.

19. A waste preparation installation having
- a first, mechanical preparation stage for separating out coarse material, iron-containing metals, foreign materials, minerals and heavy materials,
- a stage situated downstream of the latter, which has at least one percolator (1), at least one percolate preparation installation (3) according to one of Claims 13 to 18 and at least one fermenter (2),
- a stage (6) for thermal or biological drying of the material separated out from the percolate in the percolate preparation system (3) and
- a stage for mechanical separation of materials situated downstream of the stage (6) for thermal or biological drying.

## Revendications

1. Procédé pour la préparation du percolat, en particulier comme composant d'un procédé mécanique et/ou biologique de traitement des déchets, dans lequel le percolat produit par rinçage de déchets, en particulier de déchets ménagers, est amené vers une étape de préparation (3) comprenant la séparation des matières flottantes et des sédiments (3b) et la séparation des matières fibreuses (3c), où les matières flottantes et les sédiments ainsi que les matières fibreuses sont séparés avant que le percolat ainsi préparé ne soit amené au moins vers une étape de fermentation (2) pour la transformation des composants naturellement organiques en biogaz par la fermentation, **caractérisé en ce que** dans l'étape de préparation (3), le percolat est tout d'abord alimenté vers un système de tamis à tambour (3a), pour la séparation des matières grossières, dans lequel les matières grossières d'une taille de plus d'environ 3 mm jusqu'à plus d'environ 20 mm sont évacuées.

2. Procédé selon la revendication 1, **caractérisé en ce qu'**au cours de la séparation des matières grossières (3a), les composants du percolat d'une taille de plus d'environ 6 mm sont tamisées.

3. Procédé selon la revendication 1 ou 2, **caractérisé en ce qu'**au cours de l'étape de préparation (3), le percolat est alimenté vers le système de tamis à tambour (3a) au moyen d'un convoyeur à spirale.

4. Procédé selon l'une des revendications précédentes, **caractérisé en ce qu'**au cours de la séparation des matières flottantes et des sédiments (3b), la boue flottante est séparée du percolat par un racleur, et les sédiments sont séparés par un piège à sable.

5. Procédé selon l'une des revendications précédentes, **caractérisé en ce qu'**au cours de la séparation des matières fibreuses (3c), le percolat est alimenté vers un système de tamis à tambour avec une ouverture de maille effective d'environ 0,2 mm à environ 1 mm, en particulier d'environ 0,5 mm.

6. Procédé selon l'une des revendications précédentes, **caractérisé en ce qu'**après la séparation des matières grossières (3a), le percolat est alimenté, en particulier par écoulement libre, vers le piège à sable (3b) avec racleur de la séparation des matières flottantes et des sédiments, suivie de la séparation des matières fibreuses (3c).

7. Procédé selon l'une des revendications précédentes, **caractérisé en ce que** l'étape de préparation (3) est précédée d'une étape pour la séparation mécanique des grosses particules d'une taille de plus d'environ 150 mm, des métaux ferreux, des substances parasites, des minéraux et/ou des matières lourdes d'avec les déchets.

8. Procédé selon l'une des revendications précédentes, **caractérisé en ce que** le percolat est produit en pulvérisant de l'eau sur les déchets.

9. Procédé selon l'une des revendications précédentes, **caractérisé en ce que** les matières séparées du percolat au cours de l'étape de préparation (3) sont égouttées mécaniquement et/ou séchées biologiquement.

10. Procédé selon la revendication 9, **caractérisé en ce que** les matières égouttées et séchées sont alimentées vers une séparation de matière mécanique.

11. Procédé selon l'une des revendications précédentes, **caractérisé en ce que** l'eau de pressage du drainage mécanique et/ou l'eau usée de l'étape de fermentation (2) et/ou de l'étape de préparation (3) sont soumises à un traitement d'eau de procédé (4) et au moins partiellement réalimentées vers le procédé pour la fabrication du percolat à partir des déchets et/ou en tant qu'eau de rinçage.

12. Procédé selon l'une des revendications précédentes, **caractérisé en ce qu'**avant la percolation, les déchets soumis au procédé présentent une masse volumique de départ d'environ 0,35 à environ 0,5 Mg/m³, et une humidité de départ d'environ 30 à 50%.

13. Système de préparation de percolat, en particulier pour l'exécution d'un procédé selon l'une des revendications précédentes, monté en aval d'un percolateur (1) d'une système de préparation de déchets et en amont d'une étape pour la transformation biologique et/ou la fermentation (2), **caractérisé en ce qu'**il comporte un premier système de tamis à tambour (3a) pour la séparation de matières grossières d'une taille de plus d'environ 6 mm, suivi d'un piège à sable (3b) lui-même suivi d'un deuxième système de tamis à tambour (3c) pour la séparation des matières fibreuses.

14. Système de préparation de percolat selon la revendication 13, **caractérisé en ce que** le premier système de tamis à tambour (3a) comporte au moins un tamis à tambour chargé de l'intérieur, pour la séparation des matières grossières d'une taille de plus d'environ 6 mm, avec une barre de pulvérisation située à l'extérieur et/ou un système de raclage, de brossage ou de peignage, pour le nettoyage de la surface extérieure du tambour.

15. Système de préparation de percolat selon la revendication 13 ou 14, **caractérisé en ce que** le piège à sable (3b) comporte au moins un piège à sable avec une aération de piège à sable, pour l'évacuation des matières organiques d'avec les sédiments, ainsi qu'un racleur pour l'évacuation des matières solides flottantes ou de la boue flottante.

16. Système de préparation de percolat selon l'une des revendications 13 à 15, **caractérisé en ce que** le deuxième système de tamis à tambour (3c) comporte au moins un tamis à tambour chargé de l'intérieur, avec une ouverture de maille effective d'environ 0,5 mm, pour la séparation des matières fibreuses, avec un dispositif de pulvérisation basse pression et/ou un dispositif de pulvérisation haute pression situé à l'extérieur.

17. Système de préparation de percolat selon l'une des revendications 13 à 16, **caractérisé en ce que** le tamis à tambour (3a) pour la séparation des matières grossières, le piège à sable (3b) et le tamis à tambour (3c) pour la séparation des matières fibreuses ne laissent pas passer les odeurs.

18. Système de préparation de percolat selon l'une des revendications 13 à 17, **caractérisé en ce qu'**il est prévu deux tamis à tambour pour la séparation des matières grossières, deux pièges à sable et deux tamis à tambour pour la séparation des matières fibreuses.

19. Système de traitement de déchets, avec
- une première étape mécanique de préparation, pour la séparation des matières grossières, des métaux ferreux, des substances parasites, des minéraux et/ou des matières lourdes,
- une étape consécutive, comportant au moins un percolateur (1), au moins un système de préparation de percolat (3) selon l'une des revendications 13 à 18, et au moins un réacteur de fermentation (2).
- une étape (6) pour le séchage thermique ou biologique des matières séparées du percolat pendant la préparation du percolat (3), et
- une étape pour la séparation de matière mécanique, consécutive à l'étape (6) pour le séchage thermique ou biologique.
